# DEMANDE DE BREVET EUROPEEN

(11) **EP 1 514 481 A1**
(43) Date de publication de la demande: **16.03.2005**
(21) Numéro de dépôt: 04292176.7
(22) Date de dépôt: 10.09.2004
(51) Int. Cl.: A23L 1/30, A23L 1/302, A23L 1/236, A61K 9/00, A23L 1/00

(54) **Utilisation de la néohespéridine dihydrochalcone dans des comprimés à croquer comprenant des vitamines et/ou minéraux**

(30) Priorité: 11.09.2003 FR 0310688
(71) Demandeur: Fenioux, Christian, 36000 Chateauroux (FR)
(72) Inventeur: Fenioux, Christian, 36000 Chateauroux (FR)
(74) Mandataire: Ahner, Francis

(57) **Abrégé**

Utilisation de la NHDC seule pour protéger les vitamines contre leur dégradation, avantageusement dans des composés à croquer.

Utilisation de la NHDC en association avec le sorbitol pour masquer le goût désagréable des vitamines et/ou minéraux.

## Description

Les vitamines et minéraux jouent un rôle majeur dans l'ensemble des mécanismes physiologiques de l'organisme humain. Une alimentation diversifiée et, notamment, riche en fruits et légumes crus devrait permettre de fournir à l'organisme l'ensemble des vitamines et minéraux nécessaires et en quantités suffisantes. Néanmoins, les habitudes alimentaires dans les pays industrialisés conduisent de plus en plus les consommateurs à réduire la consommation de fruits et de légumes au profit de fruits et légumes transformés, ce qui a pour conséquence l'apparition d'état de carences ou de subcarences en vitamines et minéraux. Toutes les études épidémiologiques récentes confirment ces états de subcarences.

Cette situation a conduit les nutritionnistes à définir les doses optimales de vitamines et minéraux à consommer chaque jour (apports nutritionnels conseillés ou apport journaliers recommandés) et, par conséquent, à recommander une consommation plus importante de fruits et de légumes mais également une supplémentation en vitamines et minéraux les plus souvent en déficit, sous forme d'aliments enrichis en vitamines et minéraux et de compléments alimentaires.

Parmi ces nombreux produits, la majorité associe des vitamines anti-oxydantes dont les 3 majeurs sont : la vitamine A (rétinol ou caroténoïdes), la vitamine E et la vitamine C. En effet, ces vitamines anti-oxydantes ont la capacité de contrôler la production des radicaux libres issus du métabolisme de l'oxygène, et dont la surproduction est responsable de très nombreux troubles, notamment au niveau cardio-vasculaire, immunité, cancer, cutané, vieillissement accéléré, maladies neuro-dégénératives, etc.

Ces vitamines peuvent être formulées sous différentes formes, principalement sous celles de comprimés, gélules, capsules molles ou sirops.

Pour des raisons de coût et de facilité de prise, les 2 formes les plus utilisées sont les comprimés (à croquer ou à avaler) et les gélules.

Les vitamines anti-oxydantes sous forme de comprimés et de gélules rencontrent systématiquement un problème : la stabilité dans le temps. En effet, leur activité anti-oxydante au plan physiologique est liée à une très forte affinité pour l'oxygène de l'air. Dans la mesure où il est techniquement et économiquement difficile de conditionner ces formes de façon à ce qu'elles ne soient pas au contact de l'air et de l'oxygène, il se produit toujours une certaine dégradation de ces vitamines, ce qui oblige non seulement à les sur titrer à la fabrication, mais également à limiter la péremption des produits correspondants.

Il est bien connu que les vitamines, en particulier les caroténoïdes se dégradent assez rapidement dans les formes galéniques.

Si la forme comprimé permet généralement une conservation acceptable des caroténoïdes sur plusieurs années, il est par contre régulièrement observé une dégradation d'environ 10 à 15 % des caroténoïdes dans les premiers mois suivants la fabrication de ces comprimés.

Afin de limiter ce problème, il existait jusqu'à présent des solutions techniques plus ou moins satisfaisantes, notamment l'ajout de substances chimiques, ayant uniquement une activité anti-oxydante au niveau de la forme galénique.

Mais l'efficacité de ces substances est parfois limitée. De plus, ces substances n'ayant qu'une seule fonction dans la formulation leur présence augmente d'autant la quantité de substance à introduire dans la formulation, diminuant ainsi la quantité de substances actives présentes. En outre ces substances sont rarement d'origine naturelle, ce qui est contraire à la demande actuelle d'utiliser le plus d'ingrédients d'origine naturelle possible.

Il a été découvert, de façon tout à fait fortuite, que l'utilisation d'un édulcorant particulier (ingrédient apportant un goût sucré à la formule) dans une formule de comprimé contenant ces vitamines, protégeait ces vitamines contre leur dégradation, et en particulier contre leur oxydation.

Cet édulcorant est la Néohespéridine DiHydroChalcone (NHDC) obtenu par hydrogénation d'un flavonoïde naturel extrait des agrumes et, notamment, du pamplemousse.

Il s'agit d'un ingrédient autorisé au plan international, référencé en Europe sous le code E 959 et reconnu GRAS (Generally Recognized As Safe) aux USA.

Cet effet était tout à fait inattendu, puisque cet édulcorant n'avait été introduit dans cette formule de comprimé qu'à des fins organoleptiques, pour apporter un goût sucré à la formulation.

Il est connu dans le brevet FR 2 113 678 que la NHDC peut être utilisée pour conserver et prolonger la qualité du parfum dans les gommes à mâcher ou produits de confiserie courants contenant des substances aromatisantes à base d'huiles essentielle. Toutefois, le mécanisme d'action à la base de cette utilisation n'a pas été élucidé par l'auteur de ce brevet.

En outre, les vitamines et en particulier les caroténoïdes différent des parfums dont la NHDC prolonge la durée, au niveau de leur structure chimique. En effet les nombreux ingrédients lipophiles d'une huile essentielle n'ont rien à voir avec la structure chimique des vitamines, les vitamines pouvant en outre être hydrophiles ou lipophiles.

Les compléments alimentaires à base de vitamines et de minéraux se présentant sous forme de gélules, de capsules molles ou de comprimés sont destinés à être avalés : ces formes doivent donc être de taille relativement modeste ; par conséquent, chaque unité renferme entre 200 et 400 mg de poudre.

Lorsque les quantités de vitamines et minéraux à supplémenter sont assez importantes, ces formes galéniques sont mal adaptées car, pour permettre un apport suffisant, il faudrait avaler au minimum 8 à 12 unités par jour, ce qui est contraignant et ne contribue pas à une consommation régulière des doses nécessaires.

Il est donc particulièrement intéressant d'avoir recours à une autre forme galénique : le comprimé à croquer. Cette forme n'étant pas destinée à être avalée, elle peut être de taille beaucoup plus importante, pouvant aller jusqu'à 4 à 5 grammes, permettant ainsi l'apport de quantités beaucoup plus élevées de vitamines et de minéraux en une seule prise.

Cette forme présente donc un réel avantage par rapport aux formes à avaler mais fait intervenir une autre contrainte, le goût, puisque les substances actives entrent directement en contact avec les récepteurs gustatifs de la cavité buccale, ce qui n'est pas le cas des autres formes.

Par conséquent la contrainte majeure de ces comprimés à croquer est de présenter un goût acceptable et même, si possible, agréable. Or, un certain nombre de vitamines présentent un goût assez désagréable, notamment les caroténoïdes (béta-carotène, lycopène, etc.), la vitamine C et la plupart des vitamines du groupe B. Il en est de même pour certains minéraux, et notamment le fer et le zinc.

Pour résoudre ces problèmes de goût des comprimés à croquer contenant des vitamines et minéraux, les galénistes disposent de plusieurs moyens technologiques :
- enrobage des substances présentant un mauvais goût par des ingrédients neutres permettant un masquage du goût. Cette technologie présente l'inconvénient d'augmenter d'autant la quantité de substance à introduire dans le comprimé et d'augmenter de façon importante le coût de ces produits. De plus, au cours de la mastication, cet enrobage est souvent partiellement détruit, réduisant d'autant l'effet de masquage de goût.
- utilisation d'arômes et/ou d'édulcorants visant à masquer le goût des vitamines et minéraux.

Les édulcorants peuvent être des sucres (saccharose, fructose, glucose, mannitol, sorbitol) mais aussi d'autres substances ayant un pouvoir édulcorant (goût sucré) plus ou moins intense. Parmi ces substances édulcorantes autres que les sucres, on peut utiliser des substances d'origine naturelle (thaumatine, NHDC) ou synthétique (acésulfame de potassium, saccharinate de sodium, cyclamate de sodium).

La difficulté principale au cours de la formulation d'un comprimé à croquer est d'identifier la dose minimale d'édulcorant permettant un masquage efficace du goût.

En effet, les sucres constituant un apport relativement important de calories (4 kcal/g) et présentant tous un pouvoir cariogène, il est souhaitable d'en réduire l'apport.

En outre, le saccharose bien qu'ayant un pouvoir sucrant élevé, possède un index glycémique (capacité à élever le glucose sanguin après ingestion) particulièrement gênant chez les sujets diabétiques, mais aussi chez toutes les personnes souhaitant contrôler leur poids.

Le sorbitol, possède un pouvoir sucrant d'environ 60 % par rapport au saccharose et un index glycémique faible, intéressant pour les catégories de personnes définies ci-dessus. Toutefois, le sorbitol seul ne permet pas de masquer le goût désagréable des actifs, en particulier des caroténoïdes.

D'autre part, les quantités d'édulcorants d'origine naturelle ou de synthèse sont limitées par la réglementation alimentaire en vigueur.

Ainsi la NHDC nécessite, lorsqu'elle est utilisée seule pour masquer le goût des actifs tels que les caroténoïdes, des doses trop importantes, doses auxquelles apparaît une amertume de cet édulcorant. De plus la réglementation européenne fixe une dose limite d'emploi de cet édulcorant qui est de 400 mg/kg pour les compléments alimentaires contenant des vitamines et / ou des minéraux sous forme de sirop ou à mâcher, ce qui n'est pas toujours suffisant pour obtenir un masquage optimal du goût des actifs.

Des essais concernant une éventuelle synergie des pouvoirs sucrant de plusieurs édulcorants ont été réalisés (Schiffman et al., Brain Research Bulletin, Vol.38, No 2, pp.105-120, 1995). Toutefois, bien que la méthode de calcul 1 semble montrer une synergie entre la NHDC et le sorbitol, cette synergie n'apparaît qu'à une dose faible (correspondant à un solution comprenant 3% de saccharose) et pas aux doses supérieures, en particulier correspondant à 1 290 mg de sorbitol pour 0,9 mg de NHDC, pour lesquelles seule une additivité entre les pouvoirs sucrant est observée.

En outre, la méthode de calcul 2 conclut à une absence de synergie aux trois doses testées.

De plus, cette étude a été réalisée sur des compositions aqueuses ne comprenant que les deux édulcorants. Ainsi, le pouvoir masquant de ces édulcorants n'a pas été testé, en particulier en ce qui concerne le goût des caroténoïdes dans des comprimés à croquer.

Or de façon surprenante, il a été découvert par les inventeurs de la présente demande que l'association du sorbitol et du NHDC dans des comprimés à croquer permettait de masquer le goût des actifs, tels que les caroténoïdes, et qu'il existait une synergie entre ces deux édulcorants dans cette utilisation.

La présente invention concerne donc l'utilisation du NHDC pour protéger les vitamines contre leur dégradation, en particulier contre leur oxydation.

Avantageusement les vitamines sont choisies parmi la vitamine A, les caroténoïdes, la vitamine C, la vitamine E, les vitamines du groupe B et leurs mélanges.

Dans un mode de réalisation avantageux, la vitamine est un caroténoïde, avantageusement du béta-carotène, du lycopène et/ou de la vitamine A.

Dans un premier mode de réalisation de l'invention, la NHDC et la ou les vitamines se trouvent dans une composition destinée à une administration par voie orale, avantageusement sous forme de comprimés à croquer, comprimés à avaler, gélules, capsules, sirop, poudre, granulés dispersibles dans l'eau etc...

Dans un deuxième mode de réalisation de l'invention, la quantité de NHDC dans la composition est comprise entre 0,01 et 0,1 % en poids.

Dans un autre mode de réalisation de l'invention, la composition comprend en outre du sorbitol.

La composition peut également comprendre des minéraux, avantageusement choisis parmi le fer, le zinc, le sélénium et leurs mélanges.

Dans un mode particulier de réalisation de l'invention, la composition se trouve sous la forme d'un comprimé à croquer comprenant 18 à 71 % en poids de sorbitol et 0,01 à 0,05 % en poids de NHDC, soit un rapport NHDC / sorbitol compris entre 0,015 % et 0,3 %. Avantageusement le comprimé à croquer comprend 53,6 % en poids de sorbitol et 0,03 % en poids de NHDC, soit un rapport NHDC / sorbitol de 0,056 %.

Dans un autre mode de réalisation de l'invention la composition comprend de la vitamine A, de la vitamine E naturelle, de la vitamine C naturelle, du béta-carotène naturel, du lycopène naturel, de l'oxyde de zinc et du sélénium naturel, avantageusement dans les proportions respectives suivantes 0,13 % en poids, 0,72 % en poids, 17,15 % en poids, 0,6 % en poids, 0,06 % en poids, 0,36 % en poids et 4,47 % en poids.

La présente invention concerne également l'utilisation du NHDC en association avec le sorbitol pour masquer le goût des vitamines et/ou minéraux de la composition, avantageusement leur mauvais goût.

En particulier la composition se trouve sous la forme d'un comprimé à croquer.

Avantageusement les vitamines sont choisies parmi la vitamine A, les caroténoïdes, la vitamine C, la vitamine E, les vitamines du groupe B et leurs mélanges. Avantageusement les minéraux, sont choisis parmi le fer, le zinc, le sélénium et leurs mélanges.

Dans un mode de réalisation avantageux, la vitamine est un caroténoïde, avantageusement du béta-carotène, du lycopène et/ou de la vitamine A.

Dans un mode particulier de réalisation de l'invention, la composition se trouve sous la forme d'un comprimé à croquer comprenant 18 à 71 % en poids de sorbitol et 0,01 à 0,05 % en poids de NHDC, soit un rapport NHDC / sorbitol compris entre 0,015 % et 0,3 %. Avantageusement le comprimé à croquer comprend 53,6 % en poids de sorbitol et 0,03 % en poids de NHDC, soit un rapport NHDC / sorbitol de 0,056 %.

Dans un autre mode de réalisation de l'invention la composition comprend de la vitamine A, de la vitamine E naturelle, de la vitamine C naturelle, du béta-carotène naturel, du lycopène naturel, de l'oxyde de zinc et du sélénium naturel, avantageusement dans les proportions respectives suivantes 0,13 % en poids, 0,72 % en poids, 17,15 % en poids, 0,6 % en poids, 0,06 % en poids, 0,36 % en poids et 4,47 % en poids.

La présente invention concerne enfin l'utilisation du NHDC dans une composition sous forme de comprimé à croquer comprenant du sorbitol, à la fois pour protéger les vitamines de la composition, en particulier les caroténoïdes, contre leur dégradation, en particulier leur oxydation, et à la fois, en association avec le sorbitol, grâce à un effet synergique entre ces deux édulcorants, pour masquer le goût des vitamines et/ou minéraux de la composition, avantageusement le goût désagréable des caroténoïdes.

Les exemples suivants sont donnés à titre indicatif non limitatifs.

### Exemple 1 : utilisation du NHDC pour protéger les vitamines contre leur dégradation.

Un complément alimentaire sous forme de comprimé à croquer de 2800mg (formule dans tableau ci-joint) est fabriqué par compression directe après mélange de ses ingrédients dans un mélangeur de poudre.

| Ingrédients | Quantité mise en oeuvre | Equivalence en actif pur | Formule % |
|---|---|---|---|
| Vitamine A | 3,53 mg | 603µg | 0,13 |
| Vitamine E naturelle | 20,16 mg | 10,08 mg | 0,72 |
| Vitamine C naturelle | 480,20 mg | 120,05 mg | 17,15 |
| béta-carotène naturel | 16,80 mg | 1,68 mg | 0,60 |
| Lycopène naturel | 1,68 mg | 84 µg | 0,06 |
| Oxyde de Zinc | 10,08 mg | 8,06mg | 0,36 |
| Sélénium naturel | 125,16 mg | 75 µg | 4,47 |
| sorbitol | 1581,30 mg | | 56,47 |
| Arôme fruits rouges | 490 mg | | 17,50 |
| Stéarate de magnésium | 70 mg | | 2,50 |
| NHDC | 1,12 mg | | 0,04 |

Un autre comprimé est fabriqué dans lequel la NHDC est remplacée par de l'aspartam (3,92 mg) et de l'acésulfam (2,8 mg).

Il a été observé, avec la formule comprenant la NHDC, qu'après un mois de conservation à température ambiante, les teneurs en béta-carotène et lycopène des comprimés restaient pratiquement inchangées alors que dans l'autre formule ne contenant pas de NHDC, une baisse de teneur de 3 à 5 % était observée.

### Exemple 2 : synergie entre la NHDC et le sorbitol pour masquer le mauvais goût

Afin de comparer l'efficacité entre plusieurs édulcorants pour masquer le mauvais goût, une étude comparative a été réalisée.

Des comprimés à croquer, dont la composition en actifs est restée constante (cf tableau ci-après), ont été formulés en utilisant des édulcorants de nature et de quantités variables.

| Vitamines et minéraux | Quantité mise en oeuvre | Equivalence en actif pur |
|---|---|---|
| Vitamine A | 3,53 mg | 603µg |
| Vitamine E naturelle | 20,16 mg | 10,08 mg |
| Vitamine C naturelle | 480,20 mg | 120,05 mg |
| béta-carotène naturel | 16,80 mg | 1,68 mg |
| Lycopène naturel | 1,68 mg | 84 µg |
| Oxyde de Zinc | 10 mg | 8,06 mg |
| Sélénium naturel | 125 mg | 75 µg |

Les premiers essais de masquage du goût désagréable des actifs ont été réalisés avec le sucre de référence, c'est-à-dire le saccharose.

A la dose maximale de saccharose permettant de réaliser un comprimé à croquer de taille acceptable, c'est-à-dire 2500 mg de saccharose pour un comprimé de 3800 mg, le masquage du goût n'était pas encore satisfaisant.

En fait, les essais ont démontré qu'il fallait environ 3500 à 4000 mg de saccharose pour avoir un masquage satisfaisant du goût, ce qui donne des comprimés trop gros.

Le sorbitol possède un pouvoir sucrant d'environ 60 % par rapport au saccharose. Donc il n'est également pas suffisant seul pour masquer le goût désagréable des actifs.

La NHDC nécessite des doses trop importantes pour masquer le goût des actifs tels que les caroténoïdes, dose à laquelle apparaît une amertume de cet édulcorant. De plus la réglementation européenne fixe une dose limite d'emploi de cet édulcorant qui est de 400 mg/kg pour les compléments alimentaires contenant des vitamines et / ou des minéraux sous forme de sirop ou à mâcher, ce qui n'est pas suffisant pour obtenir un masquage du goût des actifs.

En prenant une base de 1500 mg de sorbitol par comprimé, ce qui correspond, avec les actifs à un comprimé d'environ 2800 mg ayant une taille parfaitement adaptée à l'utilisation « à croquer », au plan théorique il fallait donc utiliser un édulcorant apportant un pouvoir sucrant équivalent à 2600 mg de saccharose (3500 mg - 1500 x 0,6).

Parmi les essais réalisés avec différents édulcorants, un résultat tout à fait surprenant a été observé avec la NHDC. En effet, le pouvoir sucrant de cet édulcorant étant d'environ 1 500 fois celui du sucre, il conviendrait d'utiliser, selon le calcul ci-dessus, 1,7 mg de NHDC par comprimé.

Or, le comprimé pesant 2800 mg, la réglementation européenne ne permet d'utiliser que 1,12 mg / comprimé, soit 34 % de moins que la dose théorique, ce qui normalement devrait donc être insuffisant pour masquer le goût des actifs.

Or, de façon tout à fait inattendue, il est apparu que ce mélange présentait le goût le meilleur parmi tous les essais réalisés, masquant parfaitement celui des actifs.

Ce phénomène ne peut s'expliquer que par une synergie du pouvoir masquant entre le sorbitol et la NHDC, permettant ainsi de mettre en oeuvre des quantités significativement plus faibles de ces deux édulcorants.

## Revendications

1. Utilisation de la NHDC pour protéger les vitamines contre leur dégradation.

2. Utilisation selon la revendication 1 **caractérisée en ce que** les vitamines sont choisies parmi la vitamine A, les caroténoïdes, la vitamine C, la vitamine E, les vitamines du groupe B et leurs mélanges.

3. Utilisation selon l'une des revendications précédentes **caractérisée en ce que** la NHDC et les vitamines se trouvent dans une composition destinée à une administration par voie orale, avantageusement sous forme de comprimés à croquer, comprimés à avaler, gélules, capsules, sirop, poudre, ou granulés dispersibles dans l'eau.

4. Utilisation selon l'une des revendications précédentes **caractérisée en ce que** la quantité de NHDC dans la composition est comprise entre 0,01 et 0,1 % en poids.

5. Utilisation selon l'une des revendications précédentes **caractérisée en ce que** la vitamine est un caroténoïde, avantageusement du béta-carotène, du lycopène et/ou de la vitamine A.

6. Utilisation selon l'une des revendications précédentes **caractérisée en ce que** la composition comprend en outre du sorbitol.

7. Utilisation selon l'une des revendications précédentes **caractérisée en ce que** la composition comprend en outre des minéraux, avantageusement choisis parmi le fer, le zinc, le sélénium et leurs mélanges.

8. Utilisation selon la revendication 6 ou 7 **caractérisée en ce que** la NHDC en association avec le sorbitol est utilisé pour masquer le goût des vitamines et/ou minéraux de la composition, avantageusement leur mauvais goût.

9. Utilisation selon l'une des revendications précédentes **caractérisée en ce que** la composition se trouve sous la forme d'un comprimé à croquer comprenant 18 à 71 % en poids de sorbitol et 0,01 à 0,05 % en poids de NHDC.

10. Utilisation selon l'une des revendications précédentes **caractérisée en ce que** la composition comprend de la vitamine A, de la vitamine E naturelle, de la vitamine C naturelle, du béta-carotène naturel, du lycopène naturel, de l'oxyde de zinc et du sélénium naturel, avantageusement dans les proportions respectives suivantes 0,13 % en poids, 0,72 % en poids, 17,15 % en poids, 0,6 % en poids, 0,06 % en poids, 0,36 % en poids et 4,47 % en poids.
